# EUROPÄISCHE PATENTANMELDUNG

(11) **EP 0 693 659 A2**
(43) Veröffentlichungstag der Anmeldung: **24.01.1996**
(21) Anmeldenummer: 95111496.6
(22) Anmeldetag: 21.07.1995
(51) Int. Cl.: F24F 3/12

(54) **Heilklimagerät**

(30) Priorität: 23.07.1994 DE 4426218
(71) Anmelder: Metrax GmbH, D-78628 Rottweil (DE)
(72) Erfinder: Bucher, Heinz, D-786 28 Rottweil (DE)
(74) Vertreter: Fleck, Hermann-Josef, Dr.-Ing.

(57) **Zusammenfassung**

Die Erfindung betrifft ein Heilklimagerät mit einem ein Gehäuseoberteil (10) und ein Gehäuseunterteil (90) aufweisenden Gehäuse, das einen ein Gebläse aufnehmenden Luftführungskanal aufweist, der über eine Lufteintrittsöffnung (11) und Luftaustrittsöffnung (12) mit der Umgebung in Verbindung steht und in dem in Luftströmungsrichtung vor dem Gebläse ein Filterelement (33) und in Luftströmungsrichtung hinter dem Gebläse eine Sprühvorrichtung angeordnet ist, die eine Flüssigkeit in Tropfenform quer zur Förderrichtung der Luft in den Luftführungskanal sprüht. Eine höhere Druckerzeugung der geförderten Luft und damit die Schaffung der Möglichkeit zum Einsatz von Filterelementen (33) hohem Wirkungsgrad wird dadurch erreicht, daß das Gebläse ein Verdichterrad (40) aufweist, das die Luft in Achsrichtung seiner Drehachse ansaugt und über seinen Umfang radial nach außen abgibt.

## Beschreibung

Die Erfindung betrifft ein Heilklimagerät mit einem ein Gehäuseoberteil und ein Gehäuseunterteil aufweisenden Gehäuse, das einen ein Gebläse aufnehmenden Luftführungskanal aufweist, der über eine Lufteintrittsöffnung und Luftaustrittsöffnung mit der Umgebung in Verbindung steht und in dem in Luftströmungsrichtung vor dem Gebläse ein Filterelement und in Luftströmungsrichtung hinter dem Gebläse eine Sprühvorrichtung angeordnet ist, die eine Flüssigkeit in Tropfenform quer zur Förderrichtung der Luft in den Luftführungskanal sprüht.

Ein derartiges Heilklimagerät ist aus der DE 35 18 456 C2 bekannt. Die Luft wird durch die mit dem Filterelement abgedeckte Lufteintrittsöffnung in den Luftführungskanal eingesaugt. Die Luftförderung wird mittels eines Querstromlüfters ermöglicht. Vor dem Eintritt in den Querstromlüfter wird die angesaugte Luft an der UV-Lampe vorbeigeleitet. Die UV-Lampe dient zur Entkeimung der Luft. Die aus dem Querstromlüfter austretende Luft wird der Sprühvorrichtung zugeleitet. Die Sprühvorrichtung vernebelt in der Regel Meerwasser in dem Luftführungskanal. Dieses wird dann von der durchgesetzten Luft aufgenommen, so daß aus der Luftaustrittsöffnung des Luftführungskanals Wasserdampf aus dem Heilklimagerät austritt und in die Umgebung abgegeben wird.

Die zwischen dem Austritt und dem Eintritt des Querstromlüfters erzeugte Druckdifferenz ist gering, so daß das Filterelement nur einen geringen Durchgangswiderstand aufweist. Damit ist auch der Wirkungsgrad des Filterelementes entsprechend gering. Fremdkörper, insbesondere Mikroorganismen, können nicht ausgefiltert werden. Eine Luftreinigung ist somit nur zum Teil möglich. Weiterhin erweist es sich als nachteilig, daß die verwendeten bekannten Querstromlüfter eine hohe Lärmemission aufweisen, so daß der Betrieb des Heilklimagerätes vor allem in Schlafräumen als störend empfunden wird. Außerdem ist wegen der vielen, in dem Luftführungskanal vorhandenen Elemente die Luftführung behindert und auch eine Reinigung des Gerätes benachteiligt.

Es ist Aufgabe der Erfindung, ein Heilklimagerät der eingangs erwähnten Art zu schaffen, bei dem eine wirkungsvolle, ungehinderte Luftförderung erzielt wird und aus der durchgesetzten Luft auch feine Partikel und Mikroorganismen ausgefiltert werden können, das eine geringe Luftemission aufweist und bei dem der Luftführungskanal leicht zugänglich ist.

Die Aufgabe der Erfindung wird dadurch gelöst, daß in dem Gehäuse ein Zwischengehäuseteil gebildet ist, daß das Zwischengehäuseteil aus einem Zwischengehäuseoberteil und einem Zwischengehäuseunterteil gebildet ist und einen Aufnahmeraum feuchtigkeitsdicht umschließt, daß in dem Aufnahmeraum die elektronischen Bauelemente untergebracht sind, daß der Luftführungskanal zwischen dem Gehäuseoberteil und dem Zwischengehäuseoberteil gebildet ist, und daß das Gebläse ein auf einem Halteabschnitt des Zwischengehäuseoberteils angeordnetes Verdichterrad aufweist, das die Luft in Achsrichtung seiner Drehachse und über seinen Umfang radial nach außen abgibt.

Hiernach ist also vorgesehen, daß zwischen dem Gehäuseoberteil und dem Gehäuseunterteil ein feuchtigkeitsdichtes Zwischengehäuseteil aus Zwischengehäuseoberteil und Zwischengehäuseunterteil gebildet ist, in dem die elektronischen Bauelemente untergebracht sind. Hierdurch sind einerseits die elektronischen Bauelemente geschützt, andererseits sind diese nicht hinderlich im Strömungskanal angeordnet, so daß die Strömung in diesem weitgehend ungehindert verläuft und eine Reinigung ohne Schwierigkeiten möglich ist. Ferner ist das Verdichterrad in dem zwischen dem Gehäuseoberteil und dem Zwischengehäuseoberteil auf einem Halteabschnitt montiert, wodurch sich insbesondere für das als Radiallüfter aufgebaute flache Gebläse eine günstige Anordnung im Luftführungskanal ergibt. Eine gute Luftförderung wird dabei bei relativ geringer Motorleistung und geringer Geräuschentwicklung erzielt.

Mit einem so ausgestalteten Verdichterrad läßt sich zwischen Gebläseauftritt und Gebläseeintritt eine hohe Druckdifferenz erzeugen. Dies ermöglicht den Einsatz von Filterelementen mit einem hohen Wirkungsgrad. Damit können auch sehr feine Staubbestandteile und insbesondere Mikroorganismen aus der angesaugten Luft ausgefiltert werden.

Weiterhin bietet das Verdichterrad den Vorteil einer geringen Lärmemission auch bei hohem Luftdurchsatz, so daß der Betrieb des Heilklimagerätes nicht als störend empfunden wird.

Nach einer vorteilhaften Ausgestaltung der Erfindung ist vorgesehen, daß das Verdichterrad von einem, eine Lufteintritts- und eine Luftaustrittsöffnung bildenden Gehäuse umgeben ist.

Das Gehäuse leitet den erzeugten Luftstrom so, daß er gerichtet der Sprühvorrichtung zugeführt wird. Das Gehäuse ist zudem schalldämmend und kann evtl. auch mit Dämmelementen zusätzlich belegt sein, so daß eine weitere Reduzierung der Lärmemission möglich ist.

Die Auswechslung der Filtermatte ist einfach dann möglich, wenn sie auf einen Rost aufgelegt ist, der in einem Luftfilterkasten gehalten ist.

Ist vorgesehen, daß die Lufteintrittsöffnung des Luftführungskanals nach oben abgedeckt ist, so daß die Luft entgegen der Schwerkraftrichtung in den Luftführungskanal eingesaugt wird, dann ist auf einfache Weise verhindert, daß bei Nichtbetrieb des Heilklimagerätes Verschmutzungen in dem Luftführungskanal in Folge von in der Umgebung absinkenden Staubteilen auftreten.

Nach einer bevorzugten Ausgestaltung der Erfindung ist vorgesehen, daß der Luftführungskanal mittels einer Trennwand in zwei Bereiche unterteilt ist, daß die Trennwand eine Ausnehmung aufweist, die die beiden Bereiche miteinander verbindet, daß der in Luftströmungsrichtung vor der Trennwand liegende erste Bereich das Verdichterrad und der in Luftströmungsrichtung hinter der Trennwand liegende zweite Bereich die UV-Lampe und die als Brauserohr ausgebildete Sprühvorrichtung aufnimmt und daß der zweite Bereich Luftaustrittsöffnungen aufweist und mit einem einen Wasservorrat aufnehmenden Gehäuseunterteil in räumlicher Verbindung steht.

Ein einfacher Aufbau des Heilklimagerätes ist dadurch gekennzeichnet, daß das Gehäuse aus einem Gehäuseoberteil, einem Gehäuseunterteil und einem Zwischengehäuseteil gebildet ist, daß das Zwischengehäuseteil aus einem Zwischengehäuseoberteil und einem Zwischengehäuseunterteil gebildet ist und einen Aufnahmeraum feuchtigkeitsdicht umschließt und daß in dem Aufnahmeraum die elektronischen Bauelemente untergebracht sind. Mit dieser Anordnung ist auf einfache Weise erreicht, daß die elektronischen Bauelemente gekapselt und feuchtigkeitsabgeschirmt gegenüber den wasserführenden Teilen des Heilklimagerätes untergebracht sind.

Eine weitere Reduzierung des Schallpegels, der von dem Heilklimagerät erzeugt wird, ist dadurch möglich, daß das von der Sprühvorrichtung versprühte Wasser im spitzen Winkel gegen eine Prallwand geleitet ist.

Das nicht von dem Luftstrom mitgeführte Wasser wird somit an der Prallwand abgefangen und anschließend wieder dem Wasservorrat im Gehäuseunterteil zugeleitet. Um zu verhindern, daß schädliche UV-Strahlungen aus dem Gehäuse des Heilklimagerätes austreten, sieht eine erfindungsgemäße Ausgestaltung vor, daß die UV-Lampe mittels einer Abdeckung überdeckt ist.

Die Erfindung wird im folgenden anhand eines in den Zeichnungen dargestellten Ausführungsbeispiels näher erläutert. Es zeigen:
- Fig. 1: in perspektivischer Ansicht ein Heilklimagerät mit einem Gehäuseoberteil und einem Gehäuseunterteil,
- Fig. 2: das Heilklimagerät nach Fig. 1 mit von dem Gehäsueoberteil abgenommenem Deckel und
- Fig. 3: das Heilklimagerät nach den Fig. 1 und 2 in Seitendarstellung und im Schnitt.

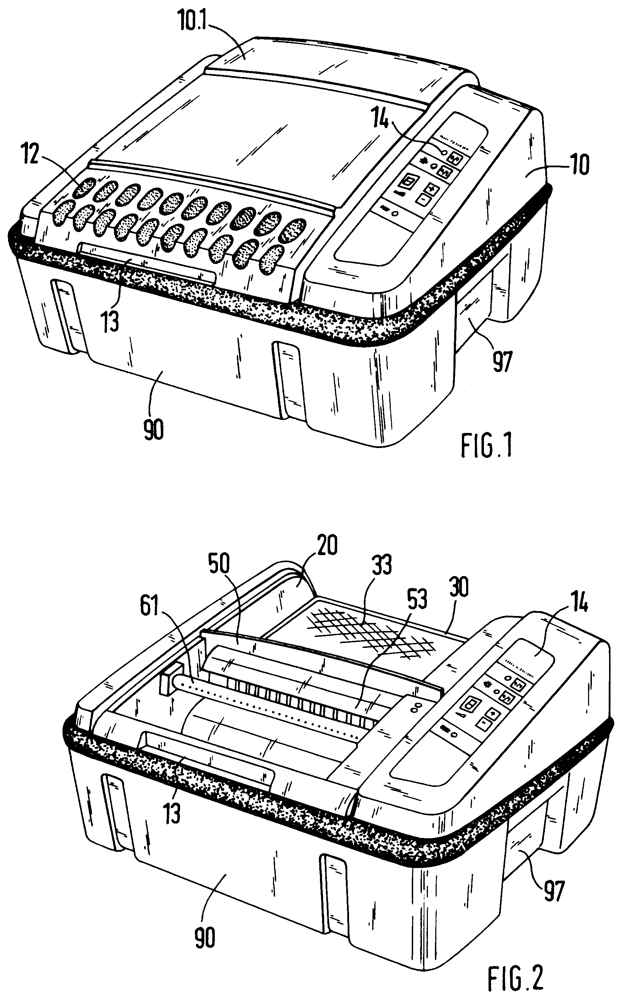

Die Fig. 1 zeigt ein Heilklimagerät mit einem Gehäuseunterteil 90 und einem Gehäuseoberteil 10. Das Gehäuseoberteil 10 weist einen Deckel 10.1 auf, der mit Luftaustrittsöffnungen 12 versehen ist. Zum Bedienen des Heilklimagerätes ist neben dem Deckel 10.1 eine Bedieneinheit 14 angeordnet. Zum Transport des Heilklimagerätes sind seitlich in das Gehäuseunterteil 90 Griffmulden 97 eingeformt. Der Deckel 10.1 läßt sich nach Aufheben einer Verriegelung 13 von dem Gehäuseobrteil 10 abheben.

Die Fig. 2 zeigt das Heilklimagerät mit abgehobenem Deckel 10.1. Wie aus dieser Darstellung ersichtlich ist, deckt der Deckel ein Filterelement 33, eine Trennwand 50 mit angeformter Abdeckung 53 sowie ein Brauserohr 61 ab. Unter der Abdeckung 53 ist eine in dieser Darstellung nicht ersichtliche UV-Lampe 60 angeordnet.

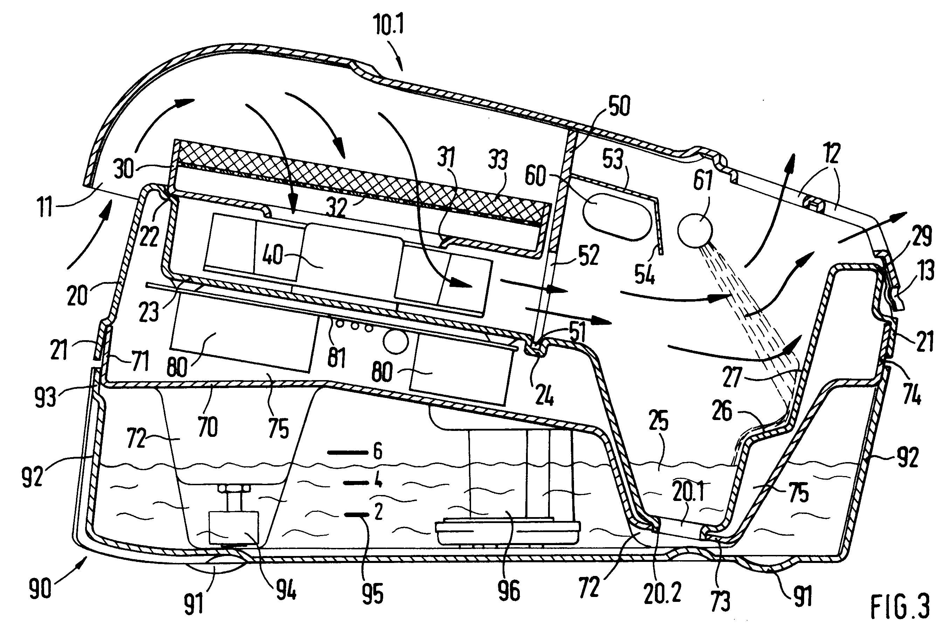

Die Fig. 3 zeigt in Seitendarstellung und im Schnitt das Heilklimagerät. Zwischen dem Gehäuseoberteil 10 und dem Gehäuseunterteil 90 ist ein Zwischengehäuse angeordnet, das im wesentlichen aus einem Zwischengehäuseoberteil 20 und einem Zwischengehäuseunterteil 70 gebildet ist. Das Zwischengehäuseoberteil 20 ist mit einem umlaufenden abgekröpften Rand 21 versehen. Mit diesem abgekröpften Rand 21 ist das Zwischengehäuseoberteil 20 auf einen nach oben abgewinkelten Rand 71 des Zwischengehäuseunterteils 70 aufgesetzt. Die Verbindung des Zwischengehäuseoberteils 20 mit dem Zwischengehäuseunterteil 70 erfolgt mittels Schraubverbindungen. Hierzu sind Befestigungsschrauben in den abgekröpften Rand 21 und den abgewinkelten Rand 71 eingeschraubt. Zwischen dem abgekröpften Rand 21 und dem abgewinkelten Rand 71 kann ein Dichtring eingelegt sein. Zwischen dem Zwischengehäuseoberteil 20 und dem Zwischengehäuseunterteil 70 ist ein Zwischenraum 75 gebildet, in dem elektrische Bauelemente 80 untergebracht sind.

Die elektrischen Bauelemente 80 sind auf einer Grundplatte 81 montiert. Die Grundplatte 81 wiederum ist mit einem Halteabschnitt 23 des Zwischengehäuseoberteils 20 verbunden. An das Zwischengehäuseunterteil 70 sind nach unten abstehende Standfüße 72 einstückig angeformt. Das aus dem Zwischengehäuseoberteil 20 und dem Zwischengehäuseunterteil 70 gebildete Zwischengehäuse ist in das Gehäuseunterteil 90 eingestellt. Hierbei stützen sich die Standfüße 72 auf dem Boden des Gehäuseunterteils 90 ab. Das Zwischengehäuse stützt sich zur Vorderseite mit seinem Zwischengehäuseunterteil 70 an einer Seitenwand 92 des Gehäuseunterteils 90 ab. Rückseitig weist die Seitenwand 92 einen abgekröpften Rand 93 auf, an dem sich das Zwischengehäuse 70 anstützt. Damit ist das Zwischengehäuse zentriert in dem Gehäuseunterteil 90 gehalten.

Das Gehäuseunterteil 90 dient zur Aufnahme eines Wasservorrates. In den den Wasservorrat aufnehmenden Bereich des Gehäuseunterteils 90 ragt eine Pumpe 96 mit ihrem Ansaugrohr. Die Pumpe 96 ist an dem Zwischengehäuseunterteil 70 festgelegt. Zur Detektierung des Wasserstandes ist ein Niveaugeber 94 verwendet, der an einem der Standfüße 72 montiert ist. Zwischen dem Deckel 10.1 des Gehäuseoberteils 10 und dem Zwischengehäuseoberteil 20 ist ein Luftführungskanal gebildet. Der Luftführungskanal steht über eine Lufteintrittsöffnung 11 und Luftaustrittsöffnung 12 mit der Umgebung in Verbindung. Die Lufteintrittsöffnung 11 ist zwischen dem Deckel 10.1 und dem Zwischengehäuseoberteil 20 gebildet. Hierzu steht der Deckel 10.1 rückseitig über das Zwischengehäuseoberteil 20 vor.

Die Lufteintrittsöffnung 11 ist nach unten geöffnet, so daß bei Nichtbetrieb des Heilklimagerätes kein Staub in den Luftführungskanal eindringen kann. Der Luftführungskanal ist mittels der Trennwand 50 in zwei Bereiche unterteilt. In dem ersten Bereich, der der Lufteintrittsöffnung 11 zugeordnet ist, ist ein ein Verdichterrad 40 aufweisendes Gebläse eingebracht. Das Gebläse ist auf dem Halteabschnitt 23 befestigt. Oberhalb des Verdichterrades 40 weist das Zwischengehäuseoberteil 20 eine Luftfilterkastenaufnahme 22 auf. Auf diese Luftfilterkastenaufnahme 22 ist ein Luftfilterkasten 30 aufgesetzt. In dem Luftfilterkasten 30 ist ein Rost 32 befestigt. Der Rost 32 trägt ein Filterelement 33. In dem zweiten, den Luftaustrittsöffnungen 12 zugeordneten Bereich ist die UV-Lampe 60 und eine als Brauserohr 61 ausgebildete Sprühvorrichtung angeordnet. Die Trennwand 50 trägt eine Abdeckung 53, die die UV-Lampe 60 überdeckt. Hierbei verhindert der Schenke 54 der Abdeckung 53, daß Licht, das von der UV-Lampe 60 ausgesandt wird, durch die Luftaustrittsöffnungen 12 des Deckels 10.1 in die Umgebung gelangt.

Unterhalb der Abdeckung 53 und der UV-Lampe 60 weist die Trennwand 50 einen Durchbruch 52 auf, mittels dem der erste und der zweite Bereich des Luftführungskanals in Verbindung stehen. Die Trennwand 50 ist mit einem Steckansatz 51 in einem Schlitz 24 des Zwischengehäuseoberteils 20 eingesetzt.

Das Gebläse saugt mit seinem Verdichterrad 40 Umgebungsluft durch die Lufteintrittsöffnung 11 an. Die angesaugte Luft tritt durch das Filterelement 33 und den Rost 32 in Achsrichtung der Drehachse in das Verdichterrad 40 ein. Im Verdichterrad 40 wird der Luftstrom um 90° umgelenkt und radial nach außen über den Umfang des Verdichterrades 40 wieder abgegeben. Die Trennwand 50, der Luftfilterkasten 30, der Halteabschnitt 23 und der an den Halteabschnitt 23 anschließende, nach oben gerichtete Steg des Zwischengehäuseoberteils 20 bilden ein Gehäuse, innerhalb dessen das Verdichterrad 40 angordnet ist. Damit wird die von dem Verdichterrad 40 ausgeblasene Luft durch den Durchbruch 52 der Trennwand 50 in den zweiten Bereich des Luftführungskanals geleitet. Nach dem Durchtritt durch den Durchbruch 52 wird die Luft mit der UV-Lampe 60 bestrahlt. Hierbei werden Keime, die mit der Luft mitgeführt werden, abgetötet. Anschließend wird die Luft durch einen von dem Brauserohr 61 erzeugten Wasservorhang hindurchgeleitet. Hierbei nimmt die Luft Feuchtigkeit auf und strömt anschließend durch die Luftaustrittsöffnung 12 wieder in die Umgebung ab.

Dem Brauserohr 61 wird das Wasser über ein Kanalsystem von der Pumpe 96 zugeführt. Das nicht von der Luft aufgenommene übrige Wasser wird von einer Prallwand 27 des Zwischengehäuseoberteils 20 abgefangen und über eine Schräge 26 nach unten abgeleitet. Von hier gelangt es in eine Vertiefung 25, die von dem Zwischengehäuseoberteil 20 gebildet ist. Die Vertiefung 25 steht über einen Durchbruch 20.1 mit dem im Gehäuseunterteil 90 aufgenommenen Wasservorrat in Verbindung. Das nicht von dem Luftstrom mitgeführte Wasser kann somit wieder in den Wasservorrat des Gehäuseunterteils 90 und steht erneut der Pumpe 96 zur Verfügung. An dem Durchbruch 20.1 ist ein ringförmiger Ansatz 20.2 angeschlossen, der in eine Ausnehmung 73 des Zwischengehäuseunterteils 70 eingesteckt ist. Der ringförmige Ansatz 20.2 ist gegen das Zwischengehäuseunterteil 70 mittels einer Ringdichtung abgedichtet. Zur Verringerung der Schallemission leitet das Brauserohr 61 das abgesprühte Wasser im spitzen Winkel gegen die Prallwand 27.

Die Reinigung des Gerätes ist einfach möglich. Hierzu muß nur der Deckel 10.1 abgenommen werden. Dies ist dadurch möglich, daß die an der Vorderseite gebildete Verriegelung 13 aufgehoben wird. Hierzu ist ein Griff vorgesehen, mittels dem der Deckel nach oben gezogen werden kann, wobei die Verriegelung 13 elastisch an der Kante 29 des Zwischengehäuseoberteils 20 zurückfedert. Bei abgenommenem Deckel 10.1 ist das Filterelement 33 zugänglich. Zum Auswechseln kann es einfach aus dem Luftfilterkasten 30 vom Rost 32 abgenommen und gegen ein neues Filterelement 33 ausgetauscht werden. Zur Reinigung des zweiten Bereiches des Luftführungskanals kann die Trennwand 50 nach oben aus dem Gehäuse herausgezogen werden. Hierdurch wird die UV-Lampe 60 freigelegt und kann mit einem Tuch gereinigt oder im Schadensfall einfach ausgetauscht werden. Das Brauserohr 61 ist herausnehmbar von oben in Aufnahmen eingesetzt. Es läßt sich einfach herausziehen und von außen und innen mit einer Bürste reinigen. Um sicherzustellen, daß nach dem erneuten Einbau des Brauserohres 61 der Sprühwinkel gegen die Prallwand 27 erhalten bleibt, sind an den das Brauserohr 61 haltenden Aufnahmen Führungen vorgesehen, die nur eine einzige Einbaulage zulassen.

Zum Nachfüllen des Wasservorrates bzw. zum Reinigen des Gehäuseunterteils 90 kann die aus Gehäuseoberteil 10, Zwischengehäuseoberteil 20 und Zwischengehäuseunterteil 70 bestehende Einheit von dem Gehäuseunterteil 90 abgehoben werden. Dann kann das Gehäuseunterteil 90 gereinigt oder Wasser aufgefüllt werden. Eine Wasserstandsanzeige 95 am Gehäuseunterteil 90 zeigt den aktuellen Füllstand.

Die UV-Lampe 60 strahlt in die Vertiefung 25 des Zwischengehäuseoberteils 25 ein. Damit bestrahlt sie auch den in dem Gehäuseunterteil 90 aufgenommenen Wasservorrat. Hierdurch wird eine Entkeimung des Wassers erzielt. Vorteilhafterweise beginnt nach dem Einschalten des Gerätes auch ein zeitlich begrenzter Entkeimungsvorgang, wobei die UV-Lampe 60 den Wasservorrat entkeimt. In diesem Betriebszustand ist das Gebläse noch nicht in Betrieb. Nach Ablauf des Entkeimungsvorganges startet das Gerät. Zur Anregung der vom Wasserdampf mitgeführten Flüssigkeitströpfchen wird ein in den Zeichnungen nicht dargestellter Schumann-Wellengenerator verwendet. Dieser erzeugt eine Schwingungsfrequenz, die sich auf die Wassertropfen überträgt. Die Frequenz einer Schumann-Welle entspricht im übrigen einer Schwingung von 7,8 Hz, die auch verstärkt in Meeresnähe meßbar ist.

Da die Schumann-Wellen sehr niederfrequent sind, muß darauf geachtet werden, daß die durch die Elektronik des Heilklimagerätes erzeugten Störimpulse die Schumann-Wellen nicht neutralisieren. Dies wird vorliegend dadurch erreicht, daß sowohl die Pumpe 96, als auch das Gebläse mit einer Null-Durchgangssteuerung betrieben werden. Für das Gebläse ist ein kommuntierter Gleichstrommotor verwendet. Hierdurch wird eine nur geringe Magnetfelderzeugung bewirkt.

## Patentansprüche

1. Heilklimagerät mit einem ein Gehäuseoberteil und ein Gehäuseunterteil aufweisenden Gehäuse, das einen ein Gebläse aufnehmenden Luftführungskanal aufweist, der über eine Lufteintrittsöffnung und Luftaustrittsöffnung mit der Umgebung in Verbindung steht und in dem in Luftströmungsrichtung vor dem Gebläse ein Filterelement und in Luftströmungsrichtung hinter dem Gebläse eine Sprühvorrichtung angeordnet ist, die eine Flüssigkeit in Tropfenform quer zur Förderrichtung der Luft in den Luftführungskanal sprüht,
dadurch gekennzeichnet,
daß in dem Gehäuse ein Zwischengehäuseteil gebildet ist,
daß das Zwischengehäuseteil aus einem Zwischengehäuseoberteil (20) und einem Zwischengehäuseunterteil (70) gebildet ist und einen Aufnahmeraum (75) feuchtigkeitsdicht umschließt,
daß in dem Aufnahmeraum (75) die elektronischen Bauelemente (80) untergebracht sind,
daß der Luftführungskanal zwischen dem Gehäuseoberteil (10) und dem Zwischengehäuseoberteil (20) gebildet ist, und
daß das Gebläse ein auf einem Halteabschnitt (23) des Zwischengehäuseoberteils (20) angeordnetes Verdichterrad (40) aufweist, das die Luft in Achsrichtung seiner Drehachse und über seinen Umfang radial nach außen abgibt.

2. Heilklimagerät nach Anspruch 1,
dadurch gekennzeichnet,
daß das Verdichterrad (40) in dem Luftführungskanal unter einem Luftfilterkasten (31) angeordnet ist.

3. Heilklimagerät nach Anspruch 1 oder 2,
dadurch gekennzeichnet,
daß die Filtermatte (33) auf einem Rost (32) aufgelegt ist, der in dem Luftfilterkasten (31) gehalten ist.

4. Heilklimagerät nach einem der Ansprüche 1 bis 3,
dadurch gekennzeichnet,
daß die Luftaustrittsöffnung (11) des Luftführungskanals nach unten gerichtet ist, so daß die Luft entgegen der Schwerkraftrichtung in den Luftführungskanal eingesaugt wird.

5. Heilklimagerät nach einem der Ansprüche 1 bis 4,
dadurch gekennzeichnet,
daß der Luftführungskanal mittels einer quer zur Luftströmungsrichtung angeordneten Trennwand (50) in zwei Bereiche unterteilt ist, daß die Trennwand (50) eine Ausnehmung (52) aufweist, die die beiden Bereiche miteinander verbindet,
daß der in Luftströmungsrichtung vor der Trennwand (50) liegende erste Bereich das Verdichterrad (40) und der in Luftströmungsrichtung hinter der Trennwand liegende zweite Bereich eine UV-Lampe (60) und die als Brausenrohr (61) ausgebildete Sprühvorrichtung aufnimmt, und
daß der zweite Bereich die Luftaustrittsöffnungen (12) aufweist und mit einem, einen Wasservorrat aufnehmenden Gehäuseunterteil (90) in räumlicher Verbindung steht.

6. Heilklimagerät nach einem der Ansprüche 1 bis 5,
dadurch gekennzeichnet,
daß das Zwischengehäuseunterteil (70) nach unten abstehende Standfüße (72) aufweist, und
daß das Zwischengehäuseteil in das Gehäuseunterteil (90) eingestellt ist.

7. Heilklimagerät nach einem der Ansprüche 1 bis 6,
dadurch gekennzeichnet,
daß die elektrischen Bauelemente (80) auf einer Grundplatte (81) montiert sind, und
daß die Grundplatte (81) mit dem Halteabschnitt (23) des Zwischengehäuseoberteils (20) verbunden ist.

8. Heilklimagerät nach einem der Ansprüche 1 bis 7,
dadurch gekennzeichnet,
daß von der Sprühvorrichtung (61) versprühtes Wasser schräg gegen die Prallwand (27) geleitet ist.

9. Heilklimagerät nach einem der Ansprüche 1 bis 8,
dadurch gekennzeichnet,
daß das Zwischengehäuseoberteil (20) in dem zweiten Bereich eine Vertiefung (25) aufweist, die in dem Zwischengehäuseoberteil (20) gebildet ist, und
daß die Vertiefung (25) mit dem Gehäuseunterteil (90) über einen Durchbruch (20.1) und einen daran angeschlossenen Ansatz (20.2) in Verbindung steht, der in eine Ausnehmung (73) des Zwischengehäuseunterteils (70) eingesteckt ist.
